# EUROPEAN PATENT APPLICATION

(11) **EP 4 834 427 A2**
(43) Date of publication of application: **04.11.2026**
(21) Application number: 26190065.8
(22) Date of filing: 06.02.2023
(51) Int. Cl.: A61P 3/06

(54) **METHODS OF WEIGHT LOSS IN A SUBJECT WITH ELEVATED HBA1C**

(30) Priority: 07.02.2022 US 202263307470 P; 04.11.2022 US 202263382426 P
(62) Document number(s) of the earlier application(s) in accordance with Art. 76 EPC: 23710591.1 / 4 475 842
(71) Applicant: Rivus Pharmaceuticals, Inc., Charlottesville, VA 22902 (US)
(72) Inventor: KHAN, Shaharyar, Charlottesville, 22901 (US); JORKASKY, Diane, Devon, 19333 (US); PORTELL, Francisco, Charlottesville, 22901 (US)
(74) Representative: D Young & Co LLP

(57) **Abstract**

The present disclosure provides a method of reducing body weight, body fat mass, liver fat in a subject who has an abnormal HbAlc level, comprising administering to the subject a therapeutically effective amount of 5-[(2,4-initrophenoxy)methyl]-1-methyl-2-nitro-1H-imidazole, or a pharmaceutically acceptable salt thereof.

## Description

### CROSS-REFERENCE TO RELATED APPLICATIONS

This International PCT Application claims priority to and the benefit of U.S. Provisional Application No. 63/307,470 filed on February 7, 2022 and U.S. Provisional Application No. 63/382,426, filed on November 4, 2022, the contents of which are herein incorporated by reference in their entirety.

### TECHNICAL FIELD

The present disclosure provides a method of reducing weight, body fat mass, and liver fat in a subject who has an abnormal HbAlc level, wherein the method comprises administering to the subject a therapeutically effective amount of 5-[(2,4-initrophenoxy)methyl]-1-methyl-2-nitro-1H-imidazole, or a pharmaceutically acceptable salt thereof.

### BACKGROUND

Obesity is a well-known risk factor for the development of many common diseases such as type 2 diabetes (T2D) and non-alcoholic fatty liver disease (NAFLD). Obesity is best viewed as any degree of excess adiposity that imparts a health risk. Glycosylated hemoglobin HbAlc is a biomarker that indicates a subject's blood glucose levels and is used along with other markers to diagnose diabetes. Obesity and overweight are among many factors that cause an elevated HbA1c. An elevated HbAlc level has been associated with a higher risk of developing complications, such as heart disease, liver disease, pancreas disease, kidney diseases, etc. Therefore, there is a great need for effective treatments for reducing weight in a subject with an elevated HbA1c.

The administration of chemical uncouplers of mitochondria as a means to decrease fat deposits has been a scientific goal for many years. While there are several small molecules which uncouple mitochondrial oxidative phosphorylation, the most well-known is 2,4-dinitrophenol (DNP). Though DNP is known to uncouple with robust effect, it unfortunately is associated with an unacceptable high rate of significant adverse effects (J. Med. Toxicol. 2011 Sep; 7(3): 205-212). These adverse effects may include hyperthermia, tachycardia, diaphoresis and tachypnoea, eventually leading to death. Being a small, highly permeable, lipophilic acid, DNP is rapidly absorbed in the stomach. The high concentration rapidly distributes and uncouples immediately, producing high levels of heat in a short period of time. Thus, DNP has a small therapeutic index and is extremely dangerous in overdose. DNP was labelled as "extremely dangerous and not fit for human consumption" by the Federal Food, Drug and Cosmetic Act of 1938. Accordingly, there is a need for uncouplers that can safely treat mitochondria-related disorders or conditions.

5-[(2,4-dinitrophenoxy)methyl]-1-methyl-2-nitro-1H-imidazole is a novel small molecule uncoupler (Compound 1). It works as a controlled metabolic accelerator (CMA). It is designed to effectively address the root cause of metabolic diseases, the accumulation of fat and sugars in the body. CMAs work to improve cellular metabolism and increase energy expenditure and calorie consumption, reducing the accumulation of fat. Using a new controlled and targeted approach, Compound 1 can increase mitochondrial proton leak, an ongoing process in the body that dissipates energy, and accounts for 20% - 40% of daily calories. Compound 1 leverages a mitochondrial uncoupling mechanism to increase substrate utilization.

### SUMMARY

In one aspect, the present disclosure provides a method for weight loss in a subject who has an abnormal HbA1c level, wherein the method comprises administering to the subject a therapeutically effective amount of 5-[(2,4-dinitrophenoxy)methyl]-1-methyl-2-nitro-1H-imidazole, or a pharmaceutically acceptable salt thereof.

In certain embodiments, the abnormal HbAlc level is the elevated HbA1c.

In another aspect, the present disclosure provides a method for reducing liver fat in a subject in need thereof, comprising administering to the subject a therapeutically effective amount of 5-[(2,4-dinitrophenoxy)methyl]-1-methyl-2-nitro-1H-imidazole, or a pharmaceutically acceptable salt thereof.

In certain embodiments, the method result in reduction of liver fat in the subject.

In certain embodiments, the method is to treat non-alcoholic fatty liver disease (NAFLD) in subjects with elevated liver fat.

In certain embodiments, the subject has a high body mass index (BMI).

In certain embodiments, the reduction of liver fat in the subject is at least 30% in the subject.

In certain embodiments, the reduction of liver fat is least 40% in the subject with the elevated HbAlc level.

In certain embodiments, the methods slow the progression of non-alcoholic fatty liver disease.

In certain embodiments, the subject suffers from obesity, excess body fat, diabetes, high blood pressure (hypertension), dyslipidemia, hypertriglyceridemia, acquired lipodystrophy, inherited lipodystrophy, partial lipodystrophy, or metabolic syndrome.

In certain embodiments, the subject is suffering from at least one of symptoms selected from reduced exercise tolerance, fatigue, tiredness, increased time to recover after exercise, and ankle swelling.

In certain embodiments, the subject suffers from disorders selected from non-alcoholic fatty liver disease (NAFLD) and non-alcoholic steatohepatitis (NASH).

In certain embodiments, the therapeutically effective amount of Compound 1 is from about 30mg to about 1400mg per day, from about 50mg to about 100mg per day, from about 150mg to about 600mg per day, or from 200mg to 550mg orally once daily.

In certain embodiments, the subject experiences weight loss after administration of Compound 1, wherein weight loss is greater than 5%, 10%, 20%, or 30%.

In one embodiment, the subject experiences at least one of:
i) a reduction of body weight by at least 5% or at least 30%;
ii) a reduction of blood pressure of at least 5 mmHg;
iii) a reduction of HbAlc by at least 0.5%, or by at least 1.5%.

In another embodiment, the method slows the progression of obesity, hypertension, or diabetes.

### DETAILED DESCRIPTION OF DRAWINGS

FIG. 1 shows the Phase 2 study design.
FIG. 2 shows the treatment effect across all doses in subjects with elevated HbAlc population.
FIG. 3 shows weight reduction in subjects with increased HbAlc.
FIG. 4 shows body fat change in subjects with elevated HbA1c population (Mean±SEM).
FIG. 5 shows weight loss in overall population and elevated HbAlc group.
FIG. 6 shows response rate (i.e. ≥ 30%) reduction in liver fat from the baseline to Day 61.
FIG. 7 shows absolute and relative percentage (%) change in liver fat at 150 mg, 300 mg, and 450 mg of Compound 1 from baseline to Day 61.
FIG. 8 shows the percent (%) change from baseline for liver stiffness parameters.
FIG. 9 shows reduction of glycated albumin (percent %) in overall (FAS) population.

### DETAILED DESCRIPTION

### Definitions

While various embodiments and aspects of the present invention are shown and described herein, it will be obvious to those skilled in the art that such embodiments and aspects are provided by way of example only. Numerous variations, changes, and substitutions will now occur to those skilled in the art without departing from the invention. It should be understood that various alternatives to the embodiments of the invention described herein may be employed in practicing the invention.

The section headings used herein are for organizational purposes only and are not to be construed as limiting the subject matter described. All documents, or portions of documents, cited in the application including, without limitation, patents, patent applications, articles, books, manuals, and treatises are hereby expressly incorporated by reference in their entirety for any purpose.

5-[(2,4-Dinitrophenoxy)methyl]-1-methyl-2-nitro-1H-imidazole is a novel small molecule uncoupler. It has the following structure:

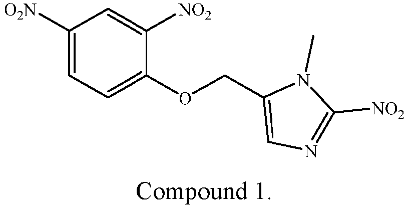

5-[(2,4-Dinitrophenoxy)methyl]-1-methyl-2-nitro-1H-imidazole may be prepared by the procedures described in WO 2018/129258.

In this disclosure, Compound 1 and CM1 are interchangeable. They both refer to 5-[(2,4-Dinitrophenoxy)methyl]-1-methyl-2-nitro-1H-imidazole.

The terms "a" or "an," as used in herein means one or more.

The terms "comprise," "include," and "have," and the derivatives thereof, are used herein interchangeably as comprehensive, open-ended terms. For example, use of "comprising," "including," or "having" means that whatever element is comprised, had, or included, is not the only element encompassed by the subject of the clause that contains the verb.

As used herein, the term "about" means a range of values including the specified value, which a person of ordinary skill in the art would consider reasonably similar to the specified value. In some embodiments, the term "about" means within a standard deviation using measurements generally acceptable in the art. In some embodiments, "about" means a range extending to +/- 10%, +/- 5%, or +/- 2% of the specified value. In some embodiments, "about" means the specified value.

As used herein, "treatment" or "treating" or "palliating" or "ameliorating" or "reducing" are used interchangeably herein. These terms refer to an approach for obtaining beneficial or desired results including but not limited to a therapeutic benefit. By therapeutic benefit means eradication or amelioration of the underlying disorder being treated. Also, a therapeutic benefit is achieved with the eradication or amelioration of one or more of the physiological symptoms associated with the underlying disorder such that an improvement is observed in the subject, notwithstanding that the subject may still be afflicted with the underlying disorder. Treatment includes causing the clinical symptoms of the disease to slow in development by administration of a composition; suppressing the disease, that is, causing a reduction in the clinical symptoms of the disease; inhibiting the disease, that is, arresting the development of clinical symptoms by administration of a composition after the initial appearance of symptoms; and/or relieving the disease, that is, causing the regression of clinical symptoms by administration of a composition after their initial appearance.

"Patient" or "subject" or "subject in need thereof" refers to a living organism suffering from or prone to a disease or condition that can be treated by using the methods provided herein. The term does not necessarily indicate that the subject has been diagnosed with a particular disease, but typically refers to an individual under medical supervision. Non-limiting examples include humans, other mammals.

As used herein, "administration" of a disclosed compound encompasses the delivery to a subject of a compound as described herein, or a prodrug or other pharmaceutically acceptable derivative thereof, using any suitable formulation or route of administration, e.g., as described herein.

"Pharmaceutically acceptable" refers to compounds, salts, compositions, dosage forms and other materials that are useful in preparing a pharmaceutical composition that is suitable for veterinary or human pharmaceutical use.

As used herein, the language "pharmaceutically acceptable salt" refers to a salt of the administered compound prepared from pharmaceutically acceptable non-toxic acids and bases, including inorganic acids, inorganic bases, organic acids, inorganic bases, solvates, hydrates, and clathrates thereof.

An "effective amount" is an amount sufficient to accomplish a stated purpose (e.g. achieve the effect for which it is administered, treat a disease, reduce enzyme activity, reduce one or more symptoms of a disease or condition, reduce viral replication in a cell). An example of an "effective amount" is an amount sufficient to contribute to the treatment, or reduction of a symptom or symptoms of a disease, which could also be referred to as a "therapeutically effective amount." A "reduction" of a symptom or symptoms (and grammatical equivalents of this phrase) means decreasing of the severity or frequency of the symptom(s), or elimination of the symptom(s). Efficacy can also be expressed as "-fold" increase or decrease. For example, a therapeutically effective amount can have at least a 1.2-fold, 1.5-fold, 2-fold, 5-fold, or more effect over a control.

As used herein, the term "increase in body temperature" in a subject refers to a body temperature increase that is associated with deleterious effects on the subject, not limited to illness, physical discomfort or pain, coma and death. In one non-limiting embodiment, the significant increase in body temperature is an increase of about 0.5° C, about 1° C, about 1.5° C, about 2° C, about 2.5° C, about 3° C, about 3.5° C, about 4° C, about 4.5° C, about 5° C, about 5.5° C, about 6° C or higher.

As used herein, "an elevated liver fat" generally refers to when more than 8% of the liver's weight is made up of fat. However, AASLD defined NAFLD elevated liver fat as 5%. Chalasani et al., Hepatology, 2018 67: 328-357. Le et al. Diabetes, 2022; 71 (Supplement_1) 119-OR. Others have suggested that any elevation of liver fat at any level is unhealthy. Minhdale et al. Diabetes 2022; 71(Supplement_1):119-OR. Other researchers suggested that the presence of any liver fat may be abnormal [and a Liver Fat Content] cutoff of around 2% may be optimal for defining non-alcoholic fatty liver disease."

### Methods of Treatment

In one aspect, provided here in is a method for weight loss in a subject who has an abnormal HbAlc level, comprising administering to the subject a therapeutically effective amount of 5-[(2,4-dinitrophenoxy)methyl]-1-methyl-2-nitro-1H-imidazole, or a pharmaceutically acceptable salt thereof.

For people without diabetes, the normal range for the hemoglobin HbAlc level is between 4% and 5.6%. Hemoglobin HbAlc levels between 5.7% and 6.4% can be characterized as prediabetes and a higher risk of developing diabetes. Levels of 6.5% or higher are considered as diabetic.

In certain embodiments, the abnormal HbAlc level is the elevated HbA1c.

In another embodiment, the subject has an elevated HbAlc level greater than 5.7.

In certain embodiments, the subject suffers from obesity, excess body fat, diabetes, high blood pressure (hypertension), dyslipidemia, hypertriglyceridemia, acquired lipodystrophy, inherited lipodystrophy, partial lipodystrophy, or metabolic syndrome.

In certain embodiments, the subject suffers from obesity or excess body fat.

In certain embodiments, the subject suffers from diabetes.

In certain embodiments, the diabetes is type 2 diabetes (T2DM).

In another embodiment, the subject suffers from disorders selected from non-alcoholic fatty liver disease (NAFLD) and non-alcoholic steatohepatitis (NASH).

In certain embodiments, the subject is suffering from at least one of symptoms selected from reduced exercise tolerance, fatigue, tiredness, increased time to recover after exercise, and ankle swelling.

In another aspect, provided herein is a method for reducing body fat mess in a subject in need thereof, comprising administering to the subject a therapeutically effective amount of 5-[(2,4-dinitrophenoxy)methyl]-1-methyl-2-nitro-1H-imidazole, or a pharmaceutically acceptable salt thereof.

In another aspect, provided herein is a method for reducing liver fat in a subject in need thereof, comprising administering to the subject a therapeutically effective amount of 5-[(2,4-dinitrophenoxy)methyl]-1-methyl-2-nitro-1H-imidazole, or a pharmaceutically acceptable salt thereof.

In certain embodiments, the subject in need thereof has elevated liver fat.

In certain embodiments, the above method result in reduction of liver fat in the subject.

In certain embodiments, the method is to treat non-alcoholic fatty liver disease (NAFLD) in subjects with elevated liver fat.

In certain embodiments, the subject has a high body mass index (BMI).

In certain embodiments, the reduction of liver fat in the subject is at least 30% in the subject.

In certain embodiments, the reduction of liver fat is least 40% in the subject with the elevated HbAlc level.

In certain embodiments, the subject's BMI is greater than 28.0 kg/m².

In certain embodiments, the subject's BMI is between 28.0 - 45.0 kg/m².

In certain embodiment, the bodyweight reduction is attributed to fat reduction.

In certain embodiment, the bodyweight reduction is attributed to liver fat reduction.

In certain embodiments, the therapeutically effective amount is 150 mg and the reduction of liver fat is about 40% in the subject.

In certain embodiments, the therapeutically effective amount is 150 mg and the reduction of liver fat is about 43% of liver fat in the subject with the elevated HbAlc level.

In certain embodiments, the therapeutically effective amount is 300 mg and the reduction of liver fat is about 70% in the subject with the elevated HbAlc level.

In certain embodiments, the therapeutically effective amount is 300 mg and the reduction of liver fat is about 75% in the subject with the elevated HbAlc level.

In certain embodiments, the therapeutically effective amount is 450 mg and the reduction of liver fat is about 72% in the subject.

In certain embodiments, the therapeutically effective amount is 450 mg and the reduction of liver fat is about 86% in the subject with the elevated HbAlc level.

In certain embodiments, the methods slow the progression of non-alcoholic fatty liver disease.

In certain embodiments, the present disclosure provides a method for reducing the risk for a subject with NAFLD to advance to non-alcoholic steatohepatitis (NASH), wherein the subjects have elevated liver fat, comprising administering to the subject a therapeutically effective amount of 5-[(2,4-dinitrophenoxy)methyl]-1-methyl-2-nitro-1H-imidazole, or a pharmaceutically acceptable salt thereof.

In another aspect, the patient with NAFLD has elevated adiposity, or elevated HbA1c.

In certain embodiments, the subject suffers from obesity, excess body fat, diabetes, high blood pressure (hypertension), dyslipidemia, hypertriglyceridemia, acquired lipodystrophy, inherited lipodystrophy, partial lipodystrophy, or metabolic syndrome.

In certain embodiments, the method slows the progression of obesity, hypertension, or diabetes.

In another aspect, disclosed here in a method for treating fibrosis, progressive fibrosis, or progressive fibrotic liver diseases NASH in a subject in need thereof, comprising administering to the subject a therapeutically effective amount of 5-[(2,4-dinitrophenoxy)methyl]-1-methyl-2-nitro-1H-imidazole, or a pharmaceutically acceptable salt thereof.

In certain embodiments, the therapeutically effective amount is from about from about 30mg to about 1400mg per day, from about 50mg to about 100mg per day, from about 150mg to about 600mg per day, or from 200mg to 550mg per day.

In certain embodiments, the therapeutically effective amount is about 100mg, 150mg, 200mg, 250mg, 300mg, 350mg, 400mg, 450mg, 500mg, or 600mg per day.

In certain embodiments, the therapeutically effective amount is about 30mg, 35mg, 40mg, 45mg, 50mg, 55mg, 60mg, 65mg, 70mg, 75mg, 80 mg, 85mg, 90mg, or 95mg, per day.

In certain embodiments, therapeutically effective amount is about 150mg, 300mg, or 450mg per day.

In certain embodiments, Compound 1 is administered orally once daily.

In certain embodiments, the subject experiences weight loss after administration of Compound 1, wherein the improvement comprising weight loss greater than 5%, 10%, >20%, or 30%.

In certain embodiments, the therapeutically effective amount of Compound 1 is about 150mg and weight loss greater than 10%.

In certain embodiments, the therapeutically effective amount of Compound 1 is about 300 mg and weight loss greater than 20%.

In certain embodiments, the therapeutically effective amount of Compound 1 is about 450 mg and weight loss greater than 30%.

In certain embodiments, the subject experiences at least one of:
i) a reduction of body weight by at least 5% or at least 30%;
ii) a reduction of blood pressure of at least 5 mmHg;
iii) a reduction of HbA1c by at least 0.5%,
iv) a reduction of lipids by at least 10%; and/or
v) a reduction of liver fat by at least 30%.

In certain embodiments, the subject experiences at least one of:
i) a reduction of body weight by at least 5% or at least 30%;
ii) a reduction of blood pressure of at least 5 mmHg;
iii) a reduction of HbA1c by at least 0.5%,
iv) a reduction of lipids by at least 10%; and/or
v) a reduction of liver fat by at least 50%.

In certain embodiments, the subject experiences a reduction of HbA1c by at least 1.5%.

In certain embodiments, the method slows the progression of obesity, hypertension, or diabetes.

### Pharmaceutical Dosage Forms

The present disclosure includes novel pharmaceutical dosage forms of Compound 1 or a pharmaceutically acceptable salt thereof. The dosage forms described herein are suitable for oral administration to a subject. The dosage form may be in any form suitable for oral administration, including, but not limited to, a capsule or a tablet. In some embodiments, the present disclosure provides a single unit dosage capsule or tablet form containing from about 30mg to about 1400mg, from about 100mg to about 1000mg, from about 150mg to about 600mg, or from 200mg to 550mg of Compound 1 or a pharmaceutically acceptable salt thereof. In some embodiments, Compound 1 is administered in a hydroxypropyl methylcellulose capsule.

In some embodiments, the amount of Compound 1 in a unit dosage is about 30mg, 40 mg, 50mg, 60 mg, 70 mg, 75mg, 80 mg, 90 mg, 100mg, 150mg, 170mg, 200mg, 250mg, 300mg, 340mg, 350mg, 400mg, 450mg, 500mg, 510mg, 550mg, 600mg, 650mg, 700mg, 750mg, 800mg, 850mg, 900mg, 950mg, 1000mg, 1050mg, 1100mg, 1150mg, 1200mg, 1250mg, 1300mg, 1350mg, or 1400mg. In some embodiments, the single unit dosage form is a capsule. In some embodiments, the single unit dosage form is a tablet.

In some embodiments, the amount of Compound 1 in a unit dosage is about 30mg, 100mg, 200mg, 500mg, 600mg, 1050mg, or 1400mg. In some embodiments, the amount of Compound 1 in a unit dosage is about 200mg, 400mg, or 550mg. In some embodiments, the amount of Compound 1 in a unit dosage is about 170mg, 340mg, 510mg. In some embodiments, the amount of Compound 1 in a unit dosage is about 150mg, 300mg, 450mg.

### Routes of Administration

In therapeutic use for controlling or preventing weight gain in a mammal, a compound of the present disclosure or its pharmaceutical compositions can be administered orally, or parenterally.

### Example 1: Phase 2a Study of Compound 1 in Subjects with Elevated Liver Fat and High Body Mass Index (BMI)

This was a 61-day randomized, double-blind trial placebo controlled trial to assess the safety and efficacy of 3 doses of orally administered Compound 1 compared to placebo in subjects with nonalcoholic fatty liver disease (NAFLD), elevated liver fat (>8%), and elevated body mass index (BMI) (28 to 45 kg/m²). Subjects were stratified by glycated hemoglobin (HbAlc ≥5.7%). The primary endpoint was the relative change in liver fat content from baseline to Day 61 assessed by magnetic resonance imaging proton density fat fraction (MRI-PDFF); secondary endpoints included safety, change in body composition, weight, glycemic control, and inflammation markers.

Eighty subjects were enrolled (placebo n=20, Compound 1 150 mg n=20, 300 mg n=21, 450 mg n=19). At baseline, HbAlc was elevated in 40% of subjects. At Day 61, the absolute and relative reductions in liver fat in Compound 1 treated subjects and the HbA1c subset were highly significant (p<0.0001 vs. placebo). A responder analysis using 30% or greater reduction in liver fat by MRI-PDFF showed responses in 40%, 71%, and 72% for Compound 1 150 mg, 300 mg, and 450 mg doses, respectively, and 43%, 75%, and 86% in the HbAlc subset vs. 0-5% with placebo (p<0.05 for all comparisons). Compound 1 treatment was associated with significant reductions in whole body fat, body weight, inflammatory markers, and glycated albumin. Lean body mass was preserved. No serious adverse events occurred. Six subjects discontinued early, 3 with Compound 1 (150 mg n=2, 300 n=1) and 3 with placebo. Diarrhea and flushing, most often mild, occurred in 35% and 25% of Compound 1 subjects, respectively.

### Study Design

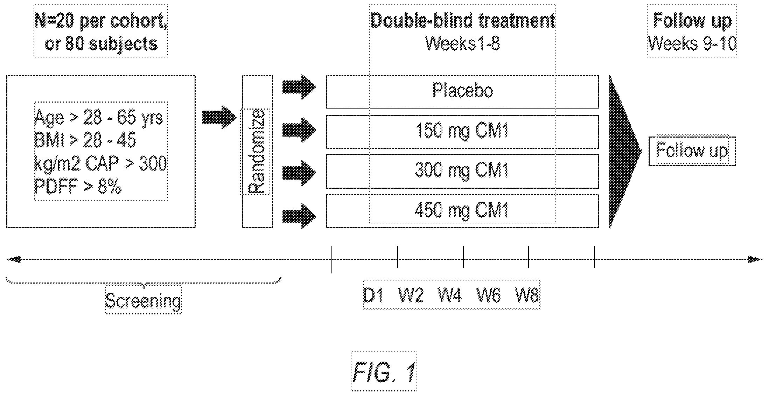

This was a Phase 2, single-center, randomized, parallel-group, double-blind, placebo-controlled, study to evaluate the safety and efficacy of Compound 1 in healthy subjects with high BMI and evidence of elevated liver fat. Subjects were screened over a 45-day period, and eligible subjects were randomized to receive once-daily oral doses of Compound 1 at 150 mg, 300 mg, or 450 mg or matching placebo under fasting conditions for 61 days. Randomization was stratified by glycated hemoglobin A1c (HbA1c) with normal baseline defined as HbAlc <5.7% and high baseline defined as HbAlc ≥5.7%. A final follow-up visit occurred within 10 to 14 days after the last dose. See Figure 1.

The randomization will be blocked and stratified by HbA1c. Subjects will be stratified into tow HbA1c strata: one subgroup of subjects with normal baseline HbAlc defined as HbAlc < 5.7% and the other subgroup of subjects with high baseline HbAlc defined as HbAlc between 5.7% and 9.0% inclusive.

### Objectives

The primary efficacy and safety objectives of this study are:

### Efficacy:

▪ To evaluate the reduction in liver fat content, as assessed by magnetic resonance imaging proton density fat fraction (MRI-PDFF), from baseline to Day 61 in subjects with elevated BMI treated with Compound 1 compared to placebo.

### Safety:

▪ To evaluate safety and tolerability of 61 days of repeated daily dosing of Compound 1 overweight and obese subjects as defined by BMI.

### The secondary objective of this study are:

▪ To assess the rate and amount of body weight loss after 61 days of Compound 1 treatment.
▪ To assess change from baseline in whole body adiposity by MRI after 61 days of Compound 1treatment.
▪ To characterize the pharmacokinetic (PK) profile of Compound 1 and its metabolities, DNP and M1, over 61 days of dosing in subjects with high BMI.
▪ To evaluate and correlate changes from baseline in measures of liver composition with changes in liver fat content after dosing with Compound 1.
▪ To investigate the pharmacodynamic (PD) effects of Compound 1 on metabolic and cardiovascular risk factors.
▪ To investigate the PD effects of Compound 1 on metabolomic, proteomic, and lipidomic profiles,
▪ To characterize the dose/exposure relationships of the efficacy and PD effects of Compound 1, as data allow.

### Inclusion Criteria

Subjects must meet all the following inclusion criteria to be eligible:
1. Adult male or females, 28 to 65 years of age (inclusive) at the time of informed consent with BMI between 28.0 and 45.0 kg/m² (inclusive).
   a. Female subjects of childbearing potential must be non-lactating, not pregnant as confirmed by a negative urine pregnancy test at Screening and agree to continue using an effective method of contraception for at least 4 weeks or barrier method for 2 weeks prior to first study drug administration until 30 days after the last dose of study drug.
   b. Female subjects of childbearing potential must not donate ova during the study and for at least 30 days after the last dose of study drug.
   c. Female subjects of non-childbearing potential must be surgically sterile (e.g., hysterectomy, bilateral tubal ligation, oophorectomy) or postmenopausal (no menses for > 1 year with follicle stimulating hormone (FSH) >40 U/L at Screening).
   d. Male subjects who have not had a vasectomy and/or subjects who have had a vasectomy but have not had 2 post surgery negative tests for sperm must agree to use an acceptable method of contraception from time of first dose of study drug until 30 days after the last dose of the study drug, and to not donate sperm during the study and for at least 30 days after the last dose of study drug.

### Exclusion Criteria

Subjects will be excluded from the study if any of the following criteria are met:
1. Insulin-controlled diabetes.
2. Pregnant or breastfeeding or plans to become pregnant.
3. Intolerance to Magnetic Resonance Imaging (MRI) or with conditions contraindicated for MRI procedures including but not limited to inability to fit into MRI scanner or surgical clips/metallic implants/shrapnel. Subjects must not be claustrophobic, have a history of claustrophobia, or intolerance of closed or small spaces.
4. Weight gain or loss >5% in 3 months prior to study or >10% in 6 months prior to screening.
5. History of lap banding, intragastric balloon, duodenal-jejunal sleeve, or bariatric surgery within 5 years of screening, plans for bariatric surgery prior to conclusion of study participation, or plans to lose weight during this study either through a special diet, exercise program or *both*.
6. History of malignant hyperthermia.
7. History of chronic serious recurrent skin rashes of unknown cause.
8. History of or current clinically significant cardiovascular disease including but not limited to transient ischemic attack, stroke, cardiac arrhythmias, syncope, unstable angina, myocardial infarction in the 6 months prior to screening, congestive heart failure, or uncontrolled hypertension. (Uncontrolled hypertension is defined as a systolic blood pressure ≥160 mmHg or a diastolic blood pressure ≥100 mmHg based on an average of three resting determinations in the sitting position with an appropriately sized cuff).
9. Resting heart rate <45 or >110 bpm.
10. On screening ECG or by history:
   a. A marked baseline prolongation of QT/QTcF interval (e.g., repeated demonstration of a QTcF interval > 450 msec for males and >470 msec for females).
   b. A history of additional risk factors for *Torsades de Pointes* (TdP) (e.g., heart failure, hypokalemia, family history of Long QT Syndrome) or a family history of sudden cardiac death of unknown origin.
11. Kidney disease, kidney transplant, or estimated glomerular filtration rate (eGFR) <50 mL/min/1.73 m² based on the CKD-EPI Creatinine Equation (NKF 2009; https://www.kidney.org/content/ckd-epi-creatinine-equation-2009).
12. Significant lung disease requiring chronic daily medication including chronic obstructive pulmonary disease (COPD), emphysema, pulmonary fibrosis, or asthma.
13. Untreated obesity hypoventilation syndrome (OHS) or obstructive sleep apnea (OSA).
14. History of or active (acute or chronic) liver disease other than nonalcoholic fatty liver disease (NAFLD)/ nonalcoholic steatohepatitis (NASH), such as but not limited to autoimmune liver disease, viral hepatitis, genetic hemochromatosis, primary biliary cirrhosis, Wilson disease, alpha-1-antitrypsin deficiency, alcohol liver disease, acute fatty liver of pregnancy or drug- induced (including acetaminophen) liver disease.
15. History of or treatment for clinically significant gastroparesis, inflammatory bowel disease, or any surgery of the upper gastrointestinal tract with the exception of cholecystectomy, or minor gastric procedures that are approved by the medical monitor.
16. History of cirrhosis and/or hepatic decompensation, including ascites, hepatic encephalopathy, or variceal bleeding.
17. History of acute pancreatitis within one year of screening or chronic pancreatitis of any cause.
18. Serum triglyceride concentrations exceeding 500 mg/dL.
19. HbAlc >9.0%.
20. Familial (mother/father/sibling) and/or personal history of retinal detachment any time in the past.
21. Any history of or current diagnosis of Glaucoma.
22. Evidence of the following on screening ophthalmologic examination:
   a. Peripheral retinal pathology requiring treatment, retinal tears, or lattice that require treatment.
   b. Diabetic retinopathy with macula exudates or macula edema as shown by optical coherence tomography (OCT) and examination.
   c. Any active macular disease that affects the vision, including macula pucker (epiretinal membrane) and macular degeneration.
   d. Visually significant cataract as determined by ophthalmologist.
   e. Any previous intravitreal injection of anti-VEGF agents for macular degeneration.
   f. History of prior vitrectomy.
23. History of malignant neoplasms within 5 years of screening, except for basal cell or squamous cell skin cancer, cervical carcinoma *in situ*, or prostate cancer that is not currently or expected to require radiation therapy, chemotherapy and/or surgical interventions or to initiate hormonal treatment.
24. History of organ transplantation.
25. Received a COVID-19 vaccine less than 1 week prior to dosing (Visit 2 / Day 1) and/or plans to receive a COVID-19 vaccine during the study period.
26. History of significant drug abuse within one year prior to Screening or frequent use of soft drugs (such as marijuana) within 3 months prior to the Screening visit, or hard drugs (such as cocaine, phencyclidine [PCP], opioid derivatives including heroin, and amphetamine derivatives) within 1 year prior to screening.
27. History of alcoholism in the last 2 years or current evidence of excessive alcohol consumption as assessed by screening evaluation using the Alcohol Use Disorders Identification Test (AUDIT, Thompson 2018), and history of regular alcohol consumption exceeding approximately 14 drinks/week for men and 7 drinks/week for women [1 drink = 4 ounces (120 mL) of wine or 12 ounces (360 mL) of beer or 1 ounce (30 mL) of hard liquor] within 6 months of Screening, as determined by the Investigator.
28. Positive urine drug screen for drugs of abuse or positive phosphatidylethanol (PEth) blood test result >100 ng/mL at Screening. In instances of an exclusionary PEth value, consideration for enrollment can be provided if the principal investigator and medical monitor agree the subject's history is not consistent with alcohol abuse.
29. Current regular vaping or more than 5 cigarettes or the equivalent per week. Use of nicotine patches for smoking cessation is permitted.
30. Positive test results of hepatitis B surface antigen (HBsAg), hepatitis C virus antibody (HCV Ab), or human immunodeficiency virus (HIV1/2) antibody.
31. Neutropenia, defined as absolute neutrophil count ≤1000/µL.
32. Serum AST or ALT >5 x upper limit of normal (ULN) at screening. (One repeat test may be allowed within 7 days at the discretion of the Investigator).
33. Total bilirubin > ULN, unless due to Gilbert's syndrome or if considered normal variability in the absence of other clinically relevant liver impairment as approved by Medical Monitor.
34. International normalized ratio (INR) ≥1.3 at screening if there is other evidence of potential significant liver impairment.
35. Participation in another clinical trial at the time of screening or exposure to any investigational agent, including topical, within 30 days of screening or 5 half-lives, if half-life known.
36. No tattoo or body piercings during the course of the study. Any underlying physical or psychological medical condition that, in the opinion of the Investigator or sponsor, would make it unlikely that the subject is able comply with the study requirements or would be unable to complete the study.
37. Any condition that the investigator believes would interfere with his/her ability to provide written informed consent, comply with study instructions, or which might confound the interpretation of the study results or put the subject at undue risk.
38. Known or potential hypersensitivity to Compound 1 or its excipients.

### Prohibited Medications (Current Use):

39. Any herbal supplement, over the counter drug, mail order or prescription drug for weight loss.
40. Prescription or over the counter stimulants including: dextroamphetamine/Dexedrine, dextroamphetamine/amphetamine combination product/Adderall, or methylphenidate (Ritalin^{®}, Concerta^{®}).
41. Thiazolidinediones (TZD): pioglitazone/Actos, rosiglitazone/Avandia.
42. Glucagon-like peptide 1 (GLP1) agonists: exenatide/Byetta/Bydureon, lixisenatide/Adlyxin, liraglutide/Victoza, dulaglutide/Trulicity, semaglutide/Ozempic.
43. Sodium-glucose cotransporter-2 (SGLT2) inhibitors: canagliflozin/Invokana, dapagliflozin/Farxiga, empagliflozin/Jardiance, ertugliflozin/Steglatro.
44. Vitamin E: use of ursodiol or high dose vitamin E >400 IU/day for at least one month within in the last 6 months or started high dose vitamin E within last 3 months of screening.
45. Recent (within 3 months of screening) or current use of obeticholic acid/Ocaliva, systemic corticosteroids, methotrexate, tamoxifen, amiodarone, or long-term use of tetracyclines.
46. Warfarin, heparin, factor Xa inhibitors (dabigatran betrixaban edoxaban, apixaban, and rivaroxaban).
47. Concomitant medications that prolong the QT/QTc interval and are known to be associated with increased risk of Torsade des pointes as identified in the https://crediblemeds.org/ website list category of 'Known Risk'.
48. Products with cannabidiol (CBD).

### Phase 2a Trial Results

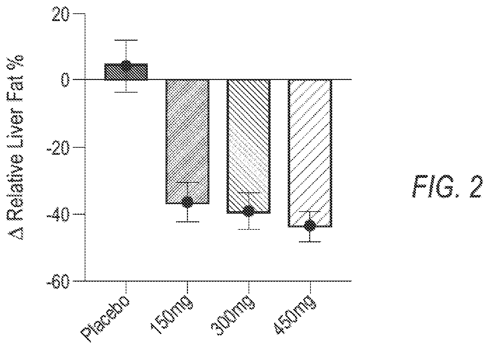

The phase 2a metabolic trial of Compound 1 was a 61-day randomized, double-blind, placebo-controlled trial designed to assess the safety and efficacy of three dose levels of Compound 1 (150 mg, 300mg, and 450 mg) in obese participants (body mass index 28 to 45 kg/m²) with elevated liver fat (greater than 8%). Eighty (80) participants ranging in age between 28 and 65 years were randomly assigned to one of three Compound 1 treatment groups or the matched placebo group, stratified and blocked for HbAlc levels of 5.7% or greater, and dosed once daily (fasting). Participants were instructed to not change behavior with regard to diet or exercise. The Phase 2a trial met primary (liver fat reduction by MRI-PDFF) and secondary (body weight and fat reduction by abdominal MRI) endpoints. Key results and observations include:
- Body weight reduction was almost exclusively from loss of fat, sparing lean body mass at all dosing levels at eight weeks, without change in diet or exercise behavior.
   ∘ Weight and fat loss was greatest at the highest dosing level, with participants losing an average of 6 pounds (p<0.001, high dose vs. placebo).
   ∘ Participants with elevated HbAlc levels experienced greater weight and fat loss, losing an average of 10 pounds (p<0.0001, high dose vs. placebo).
   ∘ Fat loss was observed in hepatic, visceral, and subcutaneous compartments by MRI.
   ∘ A >30% absolute reduction in liver fat by MRI-PDFF was observed: 40%, 71%, and 72% for the Compound 1 at 150 mg, 300 mg, and 450 mg dose levels, respectively, and 43%, 75%, and 86% with Compound 1 doses in the HbAlc subset vs. 0-5% with placebo (P<0.05 for all).
   ∘ Relative reductions in liver fat were 33%, 43%, and 40% corresponding to responder rates (>30% relative reduction) of 40%, 71% and 72% at low, mid and high doses, respectively, compared to placebo relative reduction in liver fat of 2% and responder rate of 5%.
- Compound 1 was well-tolerated at all dose levels with excellent compliance. No Serious Adverse Events or deaths were reported. Diarrhea and transient flushing associated with alcohol intake, occurring in 25% and 31.6% of Compound 1 subjects respectively, were the most commonly reported Treatment Emergent Adverse Events. The majority of these events were mild; one participant discontinued Compound 1 for diarrhea in the low dose arm while no participant discontinued for any reason at the high dose.
- Short-term treatment with Compound 1 was associated with significant improvements across primary and secondary endpoints related to NAFLD and obesity. The safety and tolerability of Compound 1 combined with the ability to reduce hepatic and whole-body adiposity in subjects regardless of HbAlc status suggest that long-term treatment with Compound 1 has the potential to be an effective treatment for NAFLD and other obesity associated metabolic diseases.
- Compound 1 at 150 mg, 300 mg, and 450 mg demonstrated significant dose-related positive effects on the primary efficacy endpoint of the change from baseline in liver fat content by MRI-PDFF across the overall population and among those with elevated HbA1c. , and these changes occurred within 61 days of treatment. The placebo-corrected change from baseline ranged from 06& to -7% with Compound 1, which compares favorably with other short-term, Phase 2 studies of drugs for NAFLD (Harrison et al, 2021; Loomba et al, 2020). Approximately 60% of the subjects overall and 68% of subjects in the subgroup experienced at least a 30% decrease in liver fat based on MRI-PDFF, and placebo-corrected mean percent change from baseline ranged from -33% to 43% for subjects overall and from -42% to -50% for subjects in the subgroup. Decreases in liver fat content were accompanied by decreases in body weight, which was accounted for by body fat without a loss of lean body mass. Improvement in liver volume, SAT, and CAP score, occurred with Compound 1, in the overall group and in the subgroup with elevated HbA1c.
   At the dose of 300 mg and 450 mg doses, Compound 1 demonstrated significant positive effects on several endpoints, including InBody scale measurements of body weight, body fat mass, and percent body fat with no significant effect on skeletal muscle mass, lean body mass or dry lean mass. Compared with placebo, mean body weight decreased by 6 pounds in the 450 mg group at Day 61 and by 10 pounds in the subgroup of subjects with elevated HbA1c, while skeletal muscle mass (and lean body mass and dry lean mass) remained unchanged. Significant reductions in inflammatory and metabolic markers were observed with Compound 1. Glycated albumin was used in this study rather than HbAlc to assess metabolic control because a change in glycated albumin occurs earlier than with HbA1c (120 days) and was a better marker of glycemic control in this 61-day study. A 0.5% reduction in HbAlc was observed, in parallel with a greater reduction in glycated albumin that was statistically significant. The preferential loss of fat and improved glycemic control in the subjects with elevated HbAlc is intriguing and as longer-term therapy has the potential to improve metabolic and inflammatory health in people with type 2 diabetes and obesity.
- The results indicate that treatment with Compound 1 improves the FAST score, and this improvement is of clinically relevant magnitude, evident in the lowest dose used (150 mg). The FAST score is a non-invasive test to identify patients with progressive liver fibrosis. Non-alcoholic fatty liver disease has a high prevalence, particularly in the obese and type 2 diabetic patient population. Some patients can live with elevated levels of liver fat without progressive disease, whereas others progress to develop NASH. Currently, a definitive diagnosis of NASH requires liver biopsy and histological scoring, an invasive and time-consuming diagnostic procedure associated with some risks. The challenges to identifying patients with progressive disease put patients at risk of progressing to later stages of NASH without intervention. Fibroscan is a non-invasive ultrasound-based measure of liver elasticity and stiffness, providing a measure of fibrosis. Aspartate aminotransferase (AST) is a liver enzyme that can be measured in a blood sample, and elevated levels indicate liver damage. In combination, Fibroscan and AST levels (FAST score) have been shown to provide a valuable indicator of patients with progressive fibrosis (Woreta et al. PLoS One 2022 April 15; 17(4): e0266859, https://doi.org/10.1371/journal.pone.0266859*.* A relatively small decrease in the FAST score of 0.2 points has been indicated to provide an improvement in several clinical measures in patients diagnosed with NASH (Wong et al, J. of Hepatology Vol. 75, pp. S257-S258) 2021; Newsome et al, The N England J of Medicine 2021;384:1113-24). Accordingly, the present disclosure provides a method for treating a subject with an elevated FAST score, wherein the subject is experiencing with progressive disease. In some embodiments, the method reduces FAST score in patients to reduce the risk of progressive disease. In some embodiments, the progressive disease includes progressive fibrosis, progress NASH, and/or progressive fibrotic liver diseases NASH.
- NASH is the form of NAFLD in which you have inflammation of the liver and liver damage, in addition to fat in your liver. The inflammation and liver damage of NASH can cause fibrosis, or scarring, of the liver. NASH may lead to cirrhosis, in which the liver is scarred and permanently damaged. Our data further indicates that Compound 1 is efficacious at lower dose levels for treating patients with HbAlc levels between 5.7% - 9.0% who are in progressive fibrotic liver diseases NASH.
- Specific efficacy and safety of Phase 2a results are shown in Figures and Tables below:
(1) Treatment effect in change relative liver fat demonstrated across all doses in subjects with elevated HbAlc as shown in Figure 2 and Table 1 below, which is the placebo-corrected percent change from baseline values for MRI-proton Density fat fraction (PDFF) in subject in HbAlc 5.7%-9.0% (Mean±SEM).

**Table 1**

| Visit | Statistic | CM1 150 mg (N=7) | CM1 300 mg (N=8) | CM1 450 mg (N=7) | Placebo (N=7) |
|---|---|---|---|---|---|
| Change from Baseline to Day 61 | n | 6 | 7 | 7 | 6 |
| | Mean | -36.488 | -39.186 | -43.734 | 4.390 |
| | SD | 14.0034 | 14.3815 | 11.9528 | 19.2118 |
| | Median | -32.985 | -39.730 | -43.100 | 4.560 |
| | Min, Max | -57.05, -18.45 | -61.21, -12.56 | -64.54, -25.14 | -22.50, 30.57 |

| | | | | | |
|---|---|---|---|---|---|
| Baseline is the last non-missing value prior to the first dose of study medication; SD stands for standard deviation. | | | | | |

(2) Analysis of covariance for MRI-proton density fat fraction (PDFF) is shown in Table 2. Mean change from baseline at Day 61 for subjects in HbAlc 5.7% -9.0%. Subgroup only (LSMean±95%CI).

**Table 2**

| Visit | Statistic | CM1 15 mg (N=7) | CM1 300 mg (N=8) | CM1 450 mg (N=7) | Placebo (N=7) |
|---|---|---|---|---|---|
| Day | LS Mean | -5.35 | -6.41 | -6.97 | 0.48 |
| 61 | 95% CI of LS Mean | (-7.64, -3.06) | (-8.48, -4.33) | (-9.02, -4.91) | (-1.86, 2.83) |
| | Difference of LS Mean and Placebo | -5.83 | -6.89 | -7.45 | |
| | 95% CI of Difference of LS Means | (-9.26, -2.40) | (-10.12, -3.66) | (-10.51, -4.39) | |
| | 2-sided P-value | 0.0020 | 0.0002 | <.0001 | |

| | | | | | |
|---|---|---|---|---|---|
| Note: This analysis was performed with the model including treatment as a fixed effect and baseline HbAlc stratification as a factor with baseline value of the response variable as a covariate Note: LS = Least Squares, CI = Confidence Interval | | | | | |

(3) Treatment results in response (>30% liver fat reduction by MRI-PDFF) across all dose arms as shown in Table 3 below:

**Table 3**

| Responder status (FAS population) | CM1 150mg (N=20) | CM1 300mg (N=21) | CM1 450mg (N=18) | All CM1 (N=59) | Placebo (N=20) |
|---|---|---|---|---|---|
| Responder | 8 (40.0%) | 15 (71.4%) | 13 (72.2%) | 36 (61.0%) | 1 (5.0%) |
| Non-responder | 10 (50.0%) | 5 (23.8%) | 5 (27.8%) | 20 (33.9%) | 16 (80.0%) |
| | | | | | |

| | 150mg CM1 (N=7) | 300mg CM1 (N=8) | 450 CM1 (N=7) | All CM1 (N=22) | Placebo (N=7) |
|---|---|---|---|---|---|
| Responder Status (HbAlc 5.7%-9.0%) | | | | | |
| Responder | 3 (42.9%) | 6 (75.0%) | 6 (85.7%) | 15 (68.2%) | 0 (0.0%) |
| Non-responder | 3 (42.9%) | 1 (12.5%) | 1 (14.3%) | 5 (22.74%) | 6 (85.7%) |

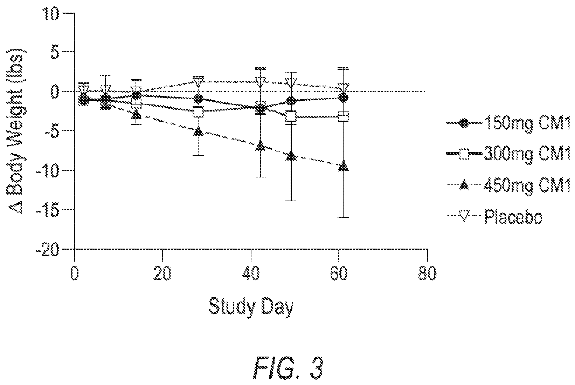

(4) Significant weight reduction (lbs) in subjects with elevated HbAlc as shown in Figure 3 and Table 4a below, which is repeated measures analysis for InBody body weight. Mean change from baseline FAS Population (LSMean±95%CI). Data for the total patient population is in Table 4b.

**Table 4a**

| Visit | Statistic | CM1 150 mg (N=7) | CM1 300 mg (N=8) | CM1 450 Mg (N=7) | Placebo (N=7) |
|---|---|---|---|---|---|
| Day 61 | LS Mean (SEM) | -0.73 (1.453) | -3.10 (1.343) | -9.35 (1.341) | 0.41 (1.466) |
| | 95% CI of LS Mean | (-3.74,2.28) | (-5.89, -0.32) | (-12.14, -6.57) | (-2.62, 3.45) |
| | Difference of LS Mean and Placebo (SEM) | -1.14 (2.066) | -3.52 (1.990) | -9.77 (1.985) | |
| | 95% CI of Difference of LS Means | (-5.42, 3.14) | (-7.64, 0.61) | (-13.89. -5.65) | |
| | 2-sided P-value | 0.5865 | 0.0907 | <.0001 | |

**Table 4b**

| Visit | Statistic | CM1 150 mg (N=20) | CM1 300 mg (N=21) | CM1 450 mg (N=18) | Placebo (N=20) |
|---|---|---|---|---|---|
| Day 61 | LS Mean (SEM) | -1.14 (1.028) | -3.92 (0.981) | -6.04 (1.034) | -0.21 (1.049) |
| | 95% CI of LS Mean | (-3.19, 0.91) | (-5.87, -1.96) | (-8.10, -3.98) | (-2.30, 1.88) |
| | Difference of LS Mean and Placebo (SEM) | -0.93 (1.467) | -3.71 (1.435) | -5.83 (1.471) | |
| | 95% CI of Difference of LS Means | (-3.86, 1.99) | (-6.57, -0.85) | (-8.77, -2.90) | |
| | 2-sided P-value | 0.5266 | 0.0117 | 0.0002 | |

| | | | | | |
|---|---|---|---|---|---|
| LS = Least Squares, SEM = Standard Error of Mean, CI = Confidence Interval. | | | | | |

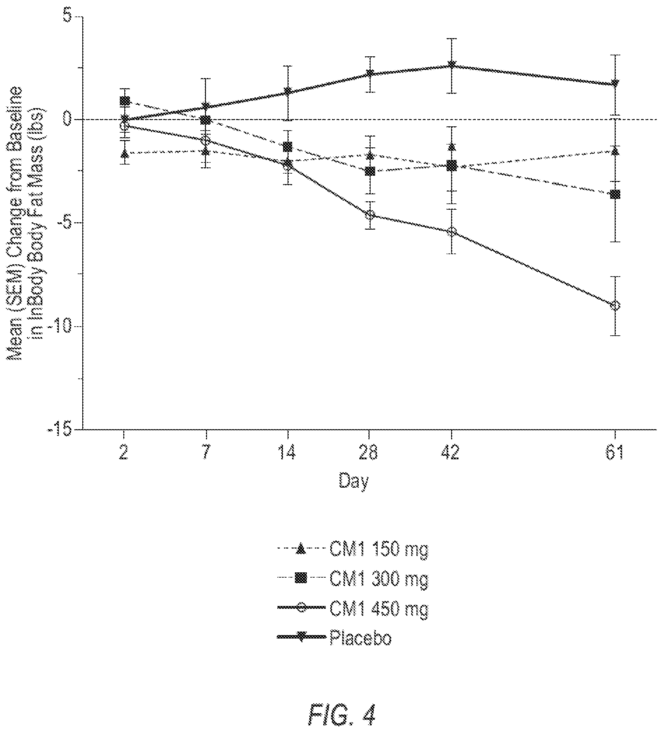

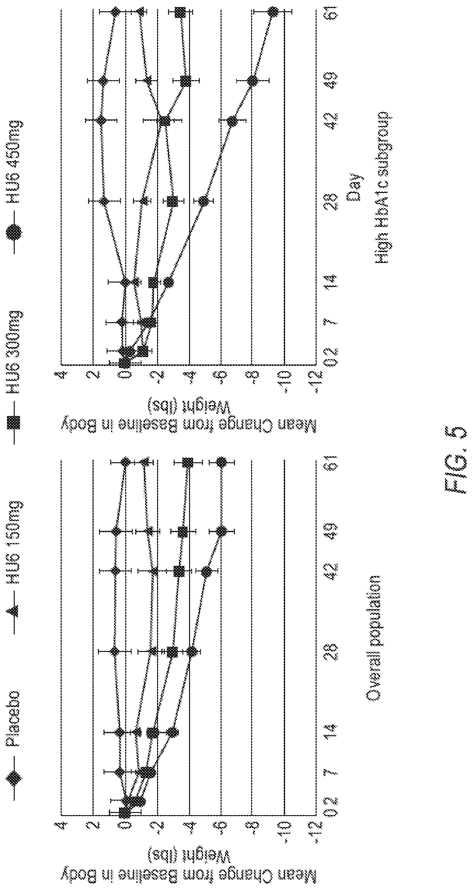

This MMRM analysis was performed with the model including the effects for treatment, visit, and treatment-by-visit interaction as fixed effects, and baseline HbAlc stratification as a factor with baseline value of the response variable as a covariate.
(5) Fat loss in subjects with elevated HbAlc population (Mean±SEM) is shown in Figure 4.
(6) Weight loss in overall population and in the elevated HbA1c group is shown in Figure 5. Percent weight changes at day 61 (using 450 mg Compound 1) is -2.55% in overall population (FAS) subjects and -4% in subjects with high HbA1c.
(7) Body Fat Mass was reduced in the elevated HbAlc group as shown in table 5 and in the overall population as shown in Table 6. The reduction in Body Fat Mass was higher in the patient population with elevated HbA1c.

**Table 5**

| Repeated Measures Analysis for Selected Secondary InBody Parameters Mean Change from Baseline for Subject s in HbAlc 5.7% - 9.0% Subgroup Only | | | | | |
|---|---|---|---|---|---|
| FAS Population | | | | | |
| Parameter: Body Fat Mass (lbs) | | | | | |
| Visit | Statistic | CM1 150 mg (N=7) | CM1 300 mg (N=8) | CM1 450 mg (N=7) | Placebo (N=7) |
| Day 61 | LS Mean (SEM) | -1.78 (1.828) | -3.36 (1.703) | -9.02 (1.696) | 1.55 (1.793) |
| | 95% CI of LS Mean | (-5.55, 2.00) | (-6.88, 0.17) | (-12.54, -5.50) | (-2.16, 5.25) |
| | Difference of LS Mean and Placebo (SEM) | -3.32 (2.562) | -4.90 (2.472) | -10.57 (2.469) | |
| | 95% CI of Difference of LS Means | (-8.61, 1.97) | (-10.01, 0.21) | (-15.67, -5.46) | |
| | 2-sided P-value | 0.2074 | 0.0593 | 0.0003 | |

**Table 6**

| Repeated Measures Analysis for Selected Secondary InBody Parameters Mean Change from Baseline FAS Population | | | | | |
|---|---|---|---|---|---|
| Parameter: Body Fat Mass (lbs) | | | | | |
| Visit | Statistic | CM1 150 mg (N=20) | CM1 300 mg (N=21) | CM1 450 mg (N=18) | Placebo (N=20) |
| Day 61 | LS Mean (SEM) | -0.42 (1.004) | -3.23 (0.961) | -5.34 (1.014) | 0.61 (1.015) |
| | 95% CI of LS Mean | (-2.42, 1.58) | (-5.15, -1.32) | (-7.37, -3.32) | (-1.41, 2.64) |
| | Difference of LS Mean and Placebo (SEM) | -1.03 (1.425) | -3.84 (1.395) | -5.95 (1.434) | |
| | 95% CI of Difference of LS Means | (-3.87, 1.81) | (-6.63, -1.06) | (-8.81, -3.09) | |
| | 2-sided P-value | 0.4715 | 0.0075 | <.0001 | |

| | | | | | |
|---|---|---|---|---|---|
| In Tables 5 and 6: LS = Least Squares, SEM = Standard Error of Mean, CI = Confidence Interval | | | | | |

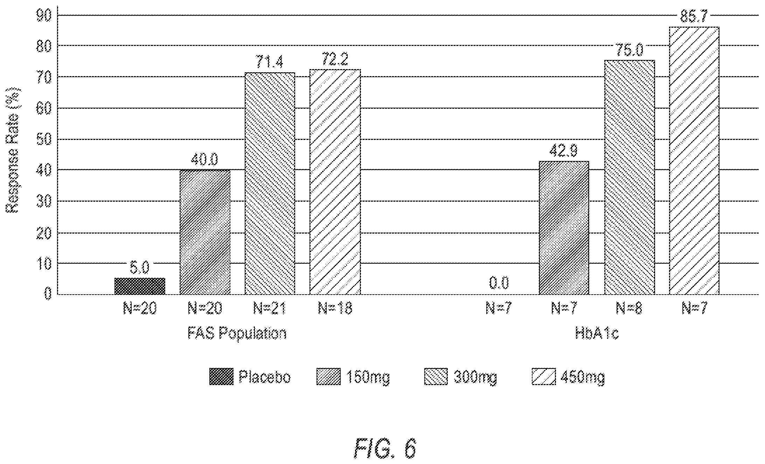

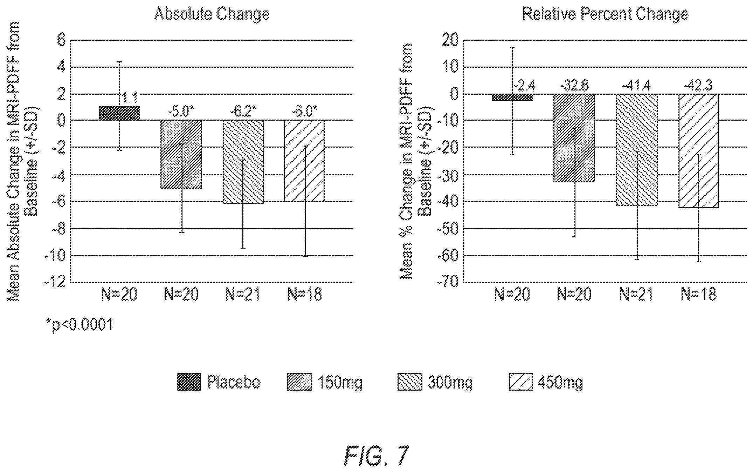

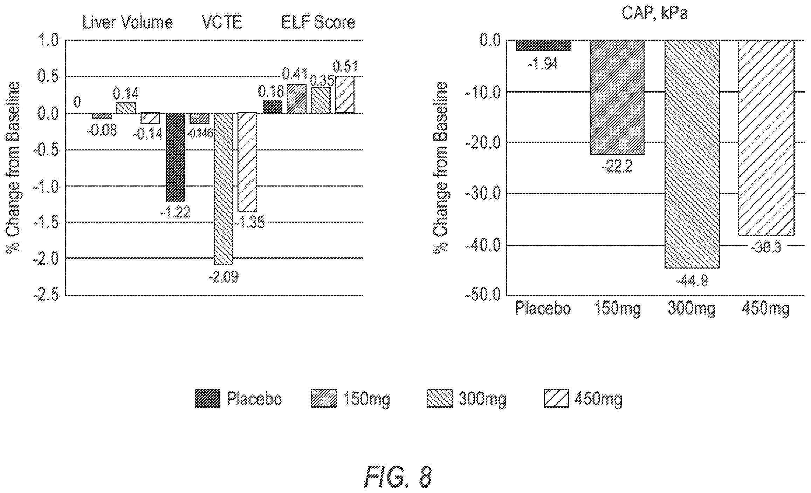

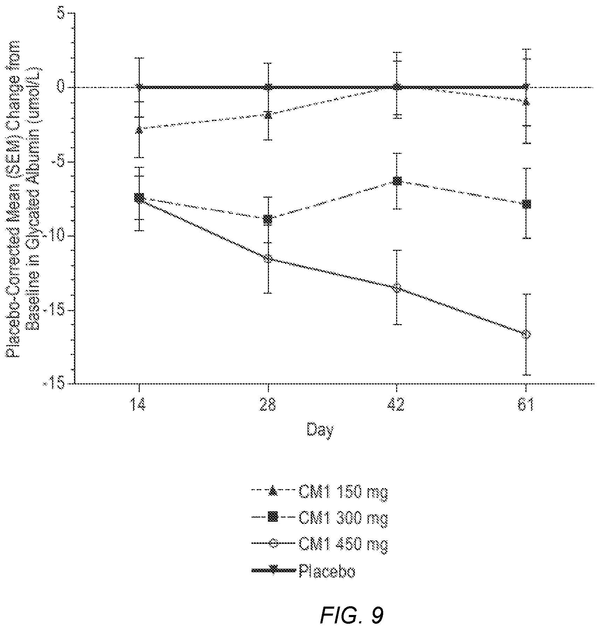

(8) Response rate, i.e. ≥30%, reduction in liver fat from the baseline to day 61 is shown in Figure 6.
(9) Absolute and relative percent (%) change in liver fat at 150 mg, 300 mg, and 450 mg of Compound 1 (from baseline to day 61) is in shown in Figure 7.
(10) Percent (%) change from baseline for liver stiffness parameters at 150 mg, 300 mg, and 450 mg of Compound 1 is shown in Figure 8.
(11) Reduction of glycated albumin (percent (%) change) from baseline to day 61 is shown in Table 7 and Figure 9.

**Table 7**

| Summary Statistics of Observed Values and Change from Baseline Values for Percent Glycated Albumin (%) by Treatment Group FAS Population | | | | | |
|---|---|---|---|---|---|
| Compound 1 Visit | Statistic | 150 mg (N=20) | 300 mg (N=21) | 450 mg (N=18) | Placebo (N=20) |
| Change from Baseline to Day 61 | n | 18 | 20 | 18 | 17 |
| | Mean | -0.18 | -0.66 | -1.49 | 0.05 |
| | SD | 1.282 | 0.994 | 0.958 | 0.655 |
| | Median | 0.1 | -0.55 | -1.25 | 0 |
| | Min, Max | -4.5, 1.7 | -2.5, 2.2 | -3.5, -0.2 | -0.7, 2.3 |
| Baseline is the last non-missing value prior to the first dose of study medication | | | | | |
| Summary Statistics of Observed Values and Change from Baseline Values for Percent Glycated Albumin (%) by Treatment Group in HbAlc 5.7% - 9.0% Subgroup Only (FAS Population) | | | | | |

| Compound 1 Visit | Statistic | 150 mg (N=7) | 300 mg (N=8) | 450 mg (N=7) | Placebo (N=7) |
|---|---|---|---|---|---|
| Change from Baseline to Day 61 | n | 6 | 7 | 7 | 6 |
| | Mean | -0.78 | -0.36 | -1.73 | 0.3 |
| | SD | 1.995 | 1.422 | 1.081 | 1.033 |
| | Median | 0.15 | -0.2 | -1.7 | 0.05 |
| | Min, Max | -4.5, 0.8 | -2.5, 2.2 | -3.5, -0.2 | -0.6, 2.3 |
| Baseline is the last non-missing value prior to the first dose of study medication | | | | | |

(12) LS mean change in liver parameters at day 61 is show in Table 8 below:

**Table 8**

| | Overall Population | | | | HbA1c Subgroup | | | |
|---|---|---|---|---|---|---|---|---|
| | PL | 150 mg | 300 mg | 450 mg | PL | 150 mg | 300 mg | 450 mg |
| Liver Volume, L | 0.00 | -0.08 | -0.14 | -0.14 | 0.00 | -0.16 | -0.17^{a} | -0.15 |
| CAP, dB/m | -1.94 | -22.2 | -44.9^{c} | -38.3^{b} | 6.37 | -33.2^{a} | -39.8^{a} | -20.0 |
| VCTE, kPa | -1.22 | -.146 | -2.09 | -1.35 | -1.78 | -1.27 | -2.84 | -0.66 |
| ELF | 0.18 | 0.41 | 0.35 | 0.51 | NA | NA | NA | NA |
| FAST | 0.031 | -0.37 | -0.319 | 0.055 | -0.018 | -0.55 | -0.69 | 0.11 |

| | | | | | | | | |
|---|---|---|---|---|---|---|---|---|
| This analysis was performed with the model including treatment as a fixed effect and baseline HbAlc stratification as a factor with baseline value of the response variable as a covariate. a p<0.05; b p<0.01; c p<0.001. | | | | | | | | |

(13) Change in MRI-Proton Density Fat Fraction (%) from baseline to Day 61 in all subjects and the subgroup of subjects with elevated HbAlc is shown in Table 9.

**Table 9**

| **Statistic** | **All Subjects** | | | | **HbA1c 5.7% to 9.0% Subgroup** | | | |
|---|---|---|---|---|---|---|---|---|
| | **150 mg (N=20)** | **300 mg (N=21)** | **450 mg (N=18)** | **Placebo (N=20)** | **150 mg (N=7)** | **300 mg (N=8)** | **450 mg (N=7)** | **Placebo (N=7)** |
| **Baseline MRI-** | | | | | | | | |
| n | 20 | 21 | 18 | 20 | 7 | 8 | 7 | 7 |
| Mean (SD) | 18.6 | 18.0 | 17.3 | 15.9 | 22.3 | 22.4 | 17.2 | 12.7 |
| Median | 16.0 | 13.9 | 13.8 | 15.0 | 22.6 | 26.0 | 14.6 | 13.8 |
| Minimum, | 9, 32 | 8, 32 | 9, 34 | 8, 27 | 9, 32 | 9, 32 | 11, 34 | 9, 16 |

| **Day 61** | | | | | | | | |
|---|---|---|---|---|---|---|---|---|
| n | 18 | 20 | 18 | 17 | 6 | 7 | 7 | 6 |
| Mean (SD) | 13.7 | 11.2 | 11.2 | 16.6 | 16.2 | 14.0 | 10.7 | 15.5 |
| Median | 12.7 | 10.4 | 8.8 | 16.1 | 19.0 | 16.1 | 8.8 | 15.7 |
| Minimum, | 6, 24 | 4, 21 | 5, 24 | 7, 32 | 6, 23 | 5, 21 | 8, 22 | 8, 22 |

| **Day 61 - Baseline^{a}** | | | | | | | | |
|---|---|---|---|---|---|---|---|---|
| n | 18 | 20 | 18 | 17 | 6 | 7 | 7 | 6 |
| Mean (SD) | -5.0 (3.09) | -6.2 (3.34) | -6.0 (4.12) | 1.1 (3.32) | -6.6 (2.69) | -7.1 (3.69) | -6.5 (4.13) | 2.1 (2.59) |
| Median | -5.8 | -5.9 | -5.1 | 1.1 | -8.0 | -9.0 | -4.7 | 1.7 |
| Minimum, maximum | -9, 2 | -11, 0 | -13, 2 | -5, 8 | -9, -3 | -11, 0 | -13, -3 | -1, 6 |
| LS Mean (95% CI)^{b} | -4.64 (-6.03, - 3.25) | -6.21 (-7.52, - 4.90) | -6.03 (-7.40, - 4.66) | 0.57 (-0.85, 1.99) | -5.35 (-7.64, - 3.06) | -6.41 (-8.48, - 4.33) | -6.97 (-9.02, - 4.91) | 0.48 (-1.86, 2.83) |

| **Difference in LS Mean (95% CI), Compound 1 - Placebo^{b}** | | | | | | | | |
|---|---|---|---|---|---|---|---|---|
| Day 61 | -5.21 (-7.19, - | -6.77 (-8.69, - | -6.60 (-8.56, - | | -5.83 (-9.26, - | -6.89 (-10.1, - | -7.45 (-10.5, - | |

| **2-sided P-value, Compound versus Placebo^{b}** | | | | | | | | |
|---|---|---|---|---|---|---|---|---|
| Day 61 | <.0001 | <.0001 | <.0001 | | 0.0020 | 0.0002 | <.0001 | |

(14) Change in liver volume and whole-body adiposity from baseline to Day 61 in all subjects and the subgroup of subjects with elevated HbAlc is shown in Table 10.

**Table 10**

| **Statistic** | | **All Subjects** | | | | **HbA1c 5.7% to 9.0% Subgroup** | | | |
|---|---|---|---|---|---|---|---|---|---|
| | | **150 mg (N=20)** | **300 mg (N=21)** | **450 mg (N=18)** | **Placebo (N=20)** | **150 mg (N=7)** | **300 mg (N=8)** | **450 mg (N=7)** | **Placebo (N=7)** |
| **Liver Volume (L)** | | | | | | | | | |
| Baseline | | | | | | | | | |
| | n | 18 | 20 | 18 | 18 | 6 | 7 | 7 | 7 |
| | Mean (SD) | 2.26 (0.521) | 2.10 (0.426) | 2.21 (0.451) | 2.19 (0.298) | 2.42 (0.526) | 2.40 (0.486) | 2.314 (0.578) | 2.26 (0.277) |
| | Median | 2.27 | 2.05 | 2.16 | 2.20 | 2.62 | 2.20 | 2.52 | 2.23 |
| | Minimum, maximum | 1.57, 3.03 | 1.53, 3.30 | 1.55, 3.00 | 1.64, 2.79 | 1.69, 3.03 | 1.79, 3.30 | 1.55, 2.96 | 1.88, 2.76 |

| Day 61 | | | | | | | | | |
|---|---|---|---|---|---|---|---|---|---|
| | n | 18 | 20 | 18 | 16 | 6 | 7 | 7 | 6 |
| | Mean (SD) | 2.18 (0.497) | 1.96 (0.459) | 2.07 (0.387) | 2.19 (0.316) | 2.26 (0.459) | 2.22 (0.587) | 2.17 (0.469) | 2.28 (0.276) |
| | Median | 2.13 | 1.87 | 2.01 | 2.12 | 2.34 | 2.01 | 2.27 | 2.13 |
| | Minimum, maximum | 1.54, 3.09 | 1.50, 3.41 | 1.57, 2.84 | 1.71, 2.76 | 1.65, 2.83 | 1.67, 3.41 | 1.57, 2.84 | 2.08, 2.76 |
| LS mean (95%CI) change from baseline at Day 61^{a, b} | | -0.08 (-0.15, 0.01) | -0.14 (-0.21, -0.08) | -0.14 (-0.21, - 0.07) | 0.00 (-0.07, 0.07) | -0.16 (-0.28, - 0.04) | -0.17 (-0.28, - 0.06) | -0.15 (-0.25, - 0.04) | 0.00 (-0.012, 0.12) |
| Difference (95% CI) in LS Mean (Compound 1 minus placebo)^{b} | | -0.08 (-0.18, 0.01) | -0.14 (-0.23, - 0.05) | -0.14 (-0.23, - 0.04) | | -0.15 (-0.32, 0.01) | -0.17 (-0.33, - 0.01) | -0.14 (-0.30, 0.02) | |
| 2-sided P-value^{b} | | 0.0956 | 0.0033 | 0.0046 | | 0.0686 | 0.0403 | 0.0780 | |

| | | | | | | | | | |
|---|---|---|---|---|---|---|---|---|---|
| Abbreviations: ANCOVA = analysis of covariance; FAS = full analysis set; HbAlc = hemoglobin A1c; LS = least squares. ^{a} Negative values indicate decreases in parameter value. ^{b} The LS means for the change from baseline and the associated 95% CIs, the difference in the LS means and the associated 95% CIs, and 2-sided p-values are from an ANCOVA model with treatment as the fixed effect, baseline HbAlc stratification as a factor, and baseline parameter value as the covariate. | | | | | | | | | |

In summary, Compound 1 at 150 mg, 300 mg, and 450 mg demonstrated significant dose-related positive effects on the primary efficacy endpoint of the change from baseline in liver fat content by MRI-PDFF across the overall population and among those with elevated HbA1c, and these changes occurred within 61 days of treatment. Approximately 60% of the subjects overall and 68% of subjects in the subgroup experienced at least a 30% decrease in liver fat based on MRI-PDFF, and placebo-corrected mean percent change from baseline ranged from -33% to -43% for subjects overall and from -42% to -50% for subjects in the subgroup. Decreases in liver fat content were accompanied by decreases in body weight, which was accounted for by body fat without a loss of lean body mass. Improvement in liver volume, SAT, and CAP score, occurred with Compound 1, in the overall group and in the subgroup with elevated HbA1c.

In addition, 300 mg and 450 mg doses of Compound 1 demonstrated significant positive effects on several secondary endpoints, including In Body scale measurements of body weight, body fat mass, and percent body fat with no effect on skeletal muscle mass, lean body mass or dry lean mass. Compared with placebo, mean body weight decreased by 6 pounds in the 450 mg group at Day 61 and by 10 pounds in the subgroup of subjects with elevated HbA1c, while skeletal muscle mass (and lean body mass and dry lean mass) remained unchanged. Significant reductions in inflammatory and metabolic markers were observed with Compound 1. Glycated albumin was used in this study rather than HbAlc to assess metabolic control because a change in glycated albumin occurs earlier than with HbAlc (120 days) and was a better marker of glycemic control in this 61-day study. A 0.5% reduction in HbAlc was observed, in parallel with a greater reduction in glycated albumin that was statistically significant. The preferential loss of fat and improved glycemic control in the subjects with elevated HbAlc is intriguing and as longer-term therapy has the potential to improve metabolic and inflammatory health in people with type 2 diabetes and obesity.
The present disclosure will now be further defined in the following paragraphs:
1. A method for weight loss in a subject who has an abnormal HbAlc level, comprising administering to the subject a therapeutically effective amount of 5-[(2,4-dinitrophenoxy)methyl]-1-methyl-2-nitro-1H-imidazole, or a pharmaceutically acceptable salt thereof.
2. A method for reduction of body fat mass in a subject who has an abnormal HbAlc level, comprising administering to the subject a therapeutically effective amount of 5-[(2,4-dinitrophenoxy)methyl]-1-methyl-2-nitro-1H-imidazole, or a pharmaceutically acceptable salt thereof.
3. The method of paragraph 1 or 2, wherein the abnormal HbAlc level is an elevated HbA1c.
4. The method of paragraph 3, wherein the subject has an elevated HbAlc level greater than 5.7.
5. A method for treating non-alcoholic fatty liver disease (NAFLD) in subjects with elevated liver fat, comprising administering to the subject a therapeutically effective amount of 5-[(2,4-dinitrophenoxy)methyl]-1-methyl-2-nitro-1H-imidazole, or a pharmaceutically acceptable salt thereof.
6. A method for reducing liver fat in a subject in need thereof, comprising administering to the subject a therapeutically effective amount of 5-[(2,4-dinitrophenoxy)methyl]-1-methyl-2-nitro-1H-imidazole, or a pharmaceutically acceptable salt thereof.
7. The method of any one of paragraphs 1-6, wherein the subject has high body mass index.
8. The method of paragraph 7, wherein the subject has body mass index greater than 28.0 kg/m².
9. The method of paragraph 6, wherein the subject has body mass index between 28.0 - 45.0 kg/m².
10. The method of any one of paragraphs 5-9, wherein the subject has an elevated HbAlc level.
11. The method of paragraph 10, wherein the subject has an elevated HbAlc level greater than 5.7.
12. The method of any one of paragraphs 6- 11, wherein the reduction of liver fat is at least 30% in the subject.
13. The method of any one of paragraphs 6-12, wherein the therapeutically effective amount is 150 mg.
14. The method of paragraph 13, wherein the method results in about 40% of liver fat reduction in the subject.
15. The method of paragraph 13, wherein the method results in about 43% of liver fat reduction in the subject with the elevated HbAlc level.
16. The method of any one of paragraphs 6-12, wherein the therapeutically effective amount is 300 mg.
17. The method of paragraph 15, wherein the method results in about 70% of liver fat reduction in the subject.
18. The method of paragraph 15, wherein the method results in about 75% of liver fat reduction in the subject with the elevated HbAlc level.
19. The method of any one of paragraphs 6-12, wherein the therapeutically effective amount is 450 mg.
20. The method of paragraph 19, wherein the method results in about 72% of liver fat in the subject.
21. The method of paragraph 19, wherein the method results in about 86% of liver fat in the subject with the elevated HbAlc level.
22. The method of any preceding paragraphs, wherein the method slows the progression of non-alcoholic fatty liver disease.
23. The method of any proceeding paragraphs, wherein the subject suffers from obesity, excess body fat, diabetes, high blood pressure (hypertension), dyslipidemia, hypertriglyceridemia, acquired lipodystrophy, inherited lipodystrophy, partial lipodystrophy, or metabolic syndrome.
24. The method of any proceeding paragraphs, wherein the subject suffers from obesity or excess body fat.
25. The method any proceeding paragraphs, wherein the subject suffers from diabetes.
26. The method of paragraph 24, wherein the diabetes is type 2 diabetes (T2DM).
27. The methods of any proceeding paragraphs, wherein the subject suffers from disorders selected from non-alcoholic fatty liver disease (NAFLD) and non-alcoholic steatohepatitis (NASH).
28. The method of any proceeding paragraphs, wherein the subject is suffering from at least one of symptoms selected from reduced exercise tolerance, fatigue, tiredness, increased time to recover after exercise, and ankle swelling.
29. A method for reduces FAST score in a subject in need thereof, comprising administering to the subject a therapeutically effective amount of 5-[(2,4-dinitrophenoxy)methyl]-1-methyl-2-nitro-1H-imidazole, or a pharmaceutically acceptable salt thereof.
30. The method of paragraph 29, wherein the method reduce the risk of progressive diseases in the subject.
31. The method of paragraph 30, wherein the progressive disease is progressive fibrosis, or progressive fibrotic liver diseases NASH.
32. A method for treating fibrosis, progressive fibrosis, or progressive fibrotic liver diseases NASH in a subject in need thereof, comprising administering to the subject a therapeutically effective amount of 5-[(2,4-dinitrophenoxy)methyl]-1-methyl-2-nitro-1H-imidazole, or a pharmaceutically acceptable salt thereof.
33. The method of any one of paragraphs 29-32, wherein the subject has high body mass index.
34. The method of paragraph 33, wherein the subject has body mass index greater than 28.0 kg/m².
35. The method of paragraph 33, wherein the subject has body mass index between 28.0 - 45.0 kg/m².
36. The method of any one of paragraphs 29-35, wherein the subject has an elevated HbA1c level.
37. The method of paragraph 36, wherein the subject has an elevated HbAlc level greater than 5.7.
38. The method of any one of paragraphs 1-12 or 29-37 wherein the therapeutically effective amount is from about 30mg to about 1400mg per day, from about 50mg to about 100mg per day, from about 150mg to about 600mg per day, or from 200mg to 550mg per day.
39. The method of any one of paragraphs 1-12 or 29-37, wherein the therapeutically effective amount is about 100mg, 150mg, 200mg, 250mg, 300mg, 350mg, 400mg, 450mg, 500mg, or 600mg per day.
40. The method of any one of paragraphs 1-12 or 29-37, wherein the therapeutically effective amount is about 150mg, 300mg, or 450mg per day.
41. The method of any one of paragraphs 1-12 or 29-37, wherein the therapeutically effective amount is about 30mg, 35mg, 40mg, 45mg, 50mg, 55mg, 60mg, 65mg, 70mg, 75mg, 80 mg, 85mg, 90mg, or 95mg, per day.
42. The method of any one of paragraphs 1-12 or 29-37, wherein 5-[(2,4-dinitrophenoxy)methyl]-1-methyl-2-nitro-1H-imidazole is administered orally once daily.
43. The method of any one of preceding paragraphs, wherein the subject experiences weight loss greater than 5%, 10%, 20%, 30%, or 40%.
44. The method of any one of preceding paragraphs, wherein the subject experiences weight loss approximately 40%.
45. The method of paragraph 34, wherein the therapeutically effective amount is about 150mg and weight loss greater than 10%.
46. The method of paragraph 34, wherein the therapeutically effective amount is about 300 mg and weight loss greater than 20%.
47. The method of paragraph 34, wherein the therapeutically effective amount is about 450 mg and weight loss greater than 30%.
48. The method of any one of preceding paragraphs 1-11, wherein the subject experiences at least one of:
   i) a reduction of body weight by at least 5% or at least 30%;
   ii) a reduction of blood pressure of at least 5 mmHg;
   iii) a reduction of HbA1c at least 0.5%;
   iv) a reduction of lipids by at least 10%; and/or
   v) a reduction of liver fat by at least 30%.
49. The method of any one of preceding paragraphs 1-11, wherein the subject experiences at least one of:
   i) a reduction of body weight by at least 5% or at least 30%;
   ii) a reduction of blood pressure of at least 5 mmHg;
   iii) a reduction of HbA1c at least 0.5%;
   iv) a reduction of lipids by at least 10%; and/or
   v) a reduction of liver fat by at least 50%.
50. The method of paragraph 48 or 49, wherein the subject experiences a reduction of HbA1c greater than 1.5%.
51. The method of any one of preceding paragraphs, wherein the method slows the progression of obesity, hypertension, or diabetes.

## Claims

1. 5-[(2,4-dinitrophenoxy)methyl]-1-methyl-2-nitro-1H-imidazole, or a pharmaceutically acceptable salt thereof for use in a method for reducing FAST score in a subject.

2. 5-[(2,4-dinitrophenoxy)methyl]-1-methyl-2-nitro-1H-imidazole, or a pharmaceutically acceptable salt thereof for the use of claim 1, wherein the subject has an elevated HbAlc.

3. 5-[(2,4-dinitrophenoxy)methyl]-1-methyl-2-nitro-1H-imidazole, or a pharmaceutically acceptable salt thereof for the use of claim 2, wherein the subject has an elevated HbAlc level greater than 5.7.

4. 5-[(2,4-dinitrophenoxy)methyl]-1-methyl-2-nitro-1H-imidazole, or a pharmaceutically acceptable salt thereof for the use of any one of claims 1-3, wherein the method reduces the risk of progressive diseases in the subject.

5. 5-[(2,4-dinitrophenoxy)methyl]-1-methyl-2-nitro-1H-imidazole, or a pharmaceutically acceptable salt thereof for the use of claim 4, wherein the progressive disease is progressive fibrosis or progressive fibrotic liver diseases NASH.

6. 5-[(2,4-dinitrophenoxy)methyl]-1-methyl-2-nitro-1H-imidazole, or a pharmaceutically acceptable salt thereof for the use of any one of claims 1-5, wherein the subject has high body mass index.

7. 5-[(2,4-dinitrophenoxy)methyl]-1-methyl-2-nitro-1H-imidazole, or a pharmaceutically acceptable salt thereof for the use of claim 6, wherein the subject has body mass index greater than 28.0 kg/m².

8. 5-[(2,4-dinitrophenoxy)methyl]-1-methyl-2-nitro-1H-imidazole, or a pharmaceutically acceptable salt thereof for the use of any one of claims 1-7, wherein 5-[(2,4-dinitrophenoxy)methyl]-1-methyl-2-nitro-1H-imidazole, or a pharmaceutically acceptable salt thereof is administered at:
A) from about 30mg to about 1400mg per day, from about 50mg to about 100mg per day, from about 150mg to about 600mg per day, or from 200mg to 550mg per day;
B) about 100mg, 150mg, 200mg, 250mg, 300mg, 350mg, 400mg, 450mg, 500mg, or 600mg per day;
C) about 150mg, 300mg, or 450mg per day; or
D) about 30mg, 35mg, 40mg, 45mg, 50mg, 55mg, 60mg, 65mg, 70mg, 75mg, 80 mg, 85mg, 90mg, or 95mg, per day.

9. 5-[(2,4-dinitrophenoxy)methyl]-1-methyl-2-nitro-1H-imidazole, or a pharmaceutically acceptable salt thereof for the use of any one of claims 1-8, wherein 5-[(2,4-dinitrophenoxy)methyl]-1-methyl-2-nitro-1H-imidazole, or a pharmaceutically acceptable salt thereof is administered orally once daily.

10. 5-[(2,4-dinitrophenoxy)methyl]-1-methyl-2-nitro-1H-imidazole, or a pharmaceutically acceptable salt thereof for the use of any one of the preceding claims, wherein the subject experiences weight loss greater than 5%, 10%, 20%, 30%, or 40%.

11. 5-[(2,4-dinitrophenoxy)methyl]-1-methyl-2-nitro-1H-imidazole, or a pharmaceutically acceptable salt thereof for the use of any one of the preceding claims, wherein the subject experiences weight loss of approximately 40%.

12. 5-[(2,4-dinitrophenoxy)methyl]-1-methyl-2-nitro-1H-imidazole, or a pharmaceutically acceptable salt thereof for the use of claim 10, wherein:
A) 5-[(2,4-dinitrophenoxy)methyl]-1-methyl-2-nitro-1H-imidazole, or a pharmaceutically acceptable salt thereof is administered at about 150mg and weight loss is greater than 10%;
B) 5-[(2,4-dinitrophenoxy)methyl]-1-methyl-2-nitro-1H-imidazole, or a pharmaceutically acceptable salt thereof is administered at about 300 mg and weight loss is greater than 20%: or
C) 5-[(2,4-dinitrophenoxy)methyl]-1-methyl-2-nitro-1H-imidazole, or a pharmaceutically acceptable salt thereof is administered at about 450 mg and weight loss is greater than 30%.

13. 5-[(2,4-dinitrophenoxy)methyl]-1-methyl-2-nitro-1H-imidazole, or a pharmaceutically acceptable salt thereof for the use of any one of the preceding claims, wherein the use slows the progression of obesity, hypertension, or diabetes.

14. 5-[(2,4-dinitrophenoxy)methyl]-1-methyl-2-nitro-1H-imidazole, or a pharmaceutically acceptable salt thereof for the use of any one of the proceeding claims, wherein the subject
A) suffers from obesity, excess body fat, diabetes, high blood pressure (hypertension), dyslipidemia, hypertriglyceridemia, acquired lipodystrophy, inherited lipodystrophy, partial lipodystrophy, or metabolic syndrome, optionally wherein the diabetes is type 2 diabetes (T2DM);
B) suffers from disorders selected from non-alcoholic fatty liver disease (NAFLD) and non-alcoholic steatohepatitis (NASH); and/or
C) is suffering from at least one of symptoms selected from reduced exercise tolerance, fatigue, tiredness, increased time to recover after exercise, and ankle swelling.
